# EUROPEAN PATENT APPLICATION

(11) **EP 1 847 618 A2**
(43) Date of publication of application: **24.10.2007**
(21) Application number: 07008000.7
(22) Date of filing: 19.04.2007
(51) Int. Cl.: C12Q 1/68

(54) **Method for detecting gastric cancer by detecting vldlr gene**

(30) Priority: 21.04.2006 JP 2006118030
(71) Applicant: FUJIFILM Corporation, Minato-ku Tokyo (JP); Tokyo Medical and Dental University, Tokyo 113-8510 (JP)
(72) Inventor: Takada, Hisashi c/o Tokyo Medical and Dental University, Tokyo 113-8510 (JP); Imoto, Issei c/o Tokyo Medical and Dental University, Tokyo 113-8510 (JP); Inazawa, Johji c/o Tokyo Medical and Dental University, Tokyo 113-8510 (JP)
(74) Representative: HOFFMANN EITLE

(57) **Abstract**

It is an objective of the present invention to provide a highly stable and reproducible method for detecting gastric cancer, by finding a novel gastric cancer-related gene and detecting the inactivation of such cancer-related gene. The present invention provides a method for detecting gastric cancer, comprising detecting malignant transformation of a gastric tissue cell by detecting the inactivation of the human VLDLR gene in the gastric tissue cell.

SEQUENCE LISTING
<110> Fuji Photo Film Co.
<120> A method for detecting stomach cancer by detection of deletion of VLDLR
gene
<130> A61300A
<160> 14
<210> 1
<211> 3646
<212> DNA
<213> Homo sapiens
<400> 1
ctctccggcc gccgccggtg cgggtgctcc gctaccggct cctctccgtt ctgtgctctc 60
ttctgctctc ggctccccac cccctctccc ttccctcctc tccccttgcc tcccctcctc 120
tgcagcgcct gcattatttt ctgcccgcag gctcggcttg cactgctgct gcagcccggg 180
gaggtggctg ggtgggtggg gaggagactg tgcaagttgt aggggagggg gtgccctctt 240
cttccccgct cccttccccc gccaactcct tcccctcctt ctcccccttt cccctccccg 300
cccccacctt cttcctcctt tcggaaggac tggtaacttg tcgtgcggag cgaacggcgg 360
cggcggcggc ggcggcggca ccatccaggc gggcaccatg ggcacgtccg cgctctgggc 420
gctctggctg ctgctcgcgc tgtgctgggc gcccccggag agcggcgcca ccggaaccgg 480
gagaaaagcc aaatgtgaac cctcccaatt ccagtgcaca aatggtcgct gtattacgct 540
gttgtggaaa tgtgatgggg atgaagactg tgttgacggc agtgatgaaa agaactgtgt 600
aaagaagacg tgtgctgaat ctgacttcgt gtgcaacaat ggccagtgtg ttcccagccg 660
atggaagtgt gatggagatc ctgactgcga agatggttca gatgaaagcc cagaacagtg 720
ccatatgaga acatgccgca tacatgaaat cagctgtggc gcccattcta ctcagtgtat 780
cccagtgtcc tggagatgtg atggtgaaaa tgattgtgac agtggagaag atgaagaaaa 840
ctgtggcaat ataacatgta gtcccgacga gttcacctgc tccagtggcc gctgcatctc 900
caggaacttt gtatgcaatg gccaggatga ctgcagcgat ggcagtgatg agctggactg 960
tgccccgcca acctgtggcg cccatgagtt ccagtgcagc acctcctcct gcatccccat 1020
cagctgggta tgcgacgatg atgcagactg ctccgaccaa tctgatgagt ccctggagca 1080

gtgtggccgt cagccagtca tacacaccaa gtgtccagcc agcgaaatcc agtgcggctc 1140
tggcgagtgc atccataaga agtggcgatg tgatggggac cctgactgca aggatggcag 1200
tgatgaggtc aactgtccct ctcgaacttg ccgacctgac caatttgaat gtgaggatgg 1260
cagctgcatc catggcagca ggcagtgtaa tggtatccga gactgtgtcg atggttccga 1320
tgaagtcaac tgcaaaaatg tcaatcagtg cttgggccct ggaaaattca agtgcagaag 1380
tggagaatgc atagatatca gcaaagtatg taaccaggag caggactgca gggactggag 1440
tgatgagccc ctgaaagagt gtcatataaa cgaatgcttg gtaaataatg gtggatgttc 1500
tcatatctgc aaagacctag ttataggcta cgagtgtgac tgtgcagctg ggtttgaact 1560
gatagatagg aaaacctgtg gagatattga tgaatgccaa aatccaggaa tctgcagtca 1620
aatttgtatc aacttaaaag gcggttacaa gtgtgaatgt agtcgtggct atcaaatgga 1680
tcttgctact ggcgtgtgca aggcagtagg caaagagcca agtctgatct tcactaatcg 1740
aagagacatc aggaagattg gcttagagag gaaagaatat atccaactag ttgaacagct 1800
aagaaacact gtggctctcg atgctgacat tgctgcccag aaactattct gggccgatct 1860
aagccaaaag gctatcttca gtgcctcaat tgatgacaag gttggtagac atgttaaaat 1920
gatcgacaat gtctataatc ctgcagccat tgctgttgat tgggtgtaca agaccatcta 1980
ctggactgat gcggcttcta agactatttc agtagctacc ctagatggaa ccaagaggaa 2040
gttcctgttt aactctgact tgcgagagcc tgcctccata gctgtggacc cactgtctgg 2100
ctttgtttac tggtcagact ggggtgaacc agctaaaata gaaaaagcag gaatgaatgg 2160
attcgataga cgtccactgg tgacagcgga tatccagtgg cctaacggaa ttacacttga 2220
ccttataaaa agtcgcctct attggcttga ttctaagttg cacatgttat ccagcgtgga 2280
cttgaatggc caagatcgta ggatagtact aaagtctctg gagttcctag ctcatcctct 2340
tgcactaaca atatttgagg atcgtgtcta ctggatagat ggggaaaatg aagcagtcta 2400
tggtgccaat aaattcactg gatcagagct agccactcta gtcaacaacc tgaatgatgc 2460
ccaagacatc attgtctatc atgaacttgt acagccatca ggtaaaaatt ggtgtgaaga 2520
agacatggag aatggaggat gtgaatacct atgcctgcca gcaccacaga ttaatgatca 2580
ctctccaaaa tatacctgtt cctgtcccag tgggtacaat gtagaggaaa atggccgaga 2640
ctgtcaaagt actgcaacta ctgtgactta cagtgagaca aaagatacga acacaacaga 2700
aatttcagca actagtggac tagttcctgg agggatcaat gtgaccacag cagtatcaga 2760
ggtcagtgtt cccccaaaag ggacttctgc cgcatgggcc attcttcctc tcttgctctt 2820

agtgatggca gcagtaggtg gctacttgat gtggcggaat tggcaacaca agaacatgaa 2880
aagcatgaac tttgacaatc ctgtgtactt gaaaaccact gaagaggacc tctccataga 2940
cattggtaga cacagtgctt ctgttggaca cacgtaccca gcaatatcag ttgtaagcac 3000
agatgatgat ctagcttgac ttctgtgaca aatgttgacc tttgaggtct aaacaaataa 3060
tacccccgtc ggaatggtaa ccgagccagc agctgaagtc tctttttctt cctctcggct 3120
ggaagaacat caagatacct ttgcgtggat caagcttgtg tacttgaccg tttttatatt 3180
acttttgtaa atattcttgt ccacattcta cttcagcttt ggatgtggtt accgagtatc 3240
tgtaaccctt gaatttctag acagtattgc cacctctggc caaatatgca ctttccctag 3300
aaagccatat tccagcagtg aaacttgtgc tatagtgtat accacctgta catacattgt 3360
ataggccatc tgtaaatatc ccagagaaca atcactattc ttaagcactt tgaaaatatt 3420
tctatgtaaa ttattgtaaa ctttttcaat ggttgggaca atggcaatag gacaaaacgg 3480
gttactaaga tgaaattgcc aaaaaaattt ataaactaat tttgtacgta tgaatgatat 3540
ctttgacctc aatggaggtt tgcaaagact gagtgttcaa actactgtac attttttttc 3600
aagtgctaaa aaattaaacc aagcagctta accatgaaaa aaaaaa 3646
<210> 2
<211> 873
<212> PRT
<213> Homo sapiens
<400> 2
<210> 3
<211> 22
<212> DNA
<213> Artificial Sequence
<220>
<223> Description of Artificial Sequence: Synthetic DNA
<400> 3
ccacagatat cagttgtaag ca                                                22
<210> 4
<211> 21
<212> DNA
<213> Artificial Sequence
<220>
<223> Description of Artificial Sequence: Synthetic DNA
<400> 4
aaaagagact tcagctgctg g                                                21

<210> 5

<211> 22

<212> DNA

<213> Artificial Sequence

<220>

<223> Description of Artificial Sequence: Synthetic DNA

<400> 5

ctagtcaaca acctgaatga tg                                                22

<210> 6

<211> 22

<212> DNA

<213> Artificial Sequence

<220>

<223> Description of Artificial Sequence: Synthetic DNA

<400> 6

aagaatggcc catgcggcag aa                                                22

<210> 7

<211> 25

<212> DNA

<213> Artificial Sequence

<220>

<223> Description of Artificial Sequence: Synthetic DNA

<400> 7

gaaattgtaa gtatttttta ggtgt                                             25

<210> 8

<211> 23

<212> DNA

<213> Artificial Sequence

<220>

<223> Description of Artificial Sequence: Synthetic DNA

<400> 8

tacctatcct aatttcctaa tcc                                                23

<210> 9

<211> 23

<212> DNA

<213> Artificial Sequence

<220>

<223> Description of Artificial Sequence: Synthetic DNA

<400> 9

ggattaggaa attaggatag gta                                                23

<210> 10

<211> 18

<212> DNA

<213> Artificial Sequence

<220>

<223> Description of Artificial Sequence: Synthetic DNA

<400> 10

cccacccaac cacctccc                                                      18

<210> 11

<211> 19

<212> DNA

<213> Artificial Sequence

<220>

<223> Description of Artificial Sequence: Synthetic DNA

<400> 11

gagttttagg cgaacgcgc                                                     19

<210> 12

<211> 18

<212> DNA
<213> Artificial Sequence
<220>
<223> Description of Artificial Sequence: Synthetic DNA
<400> 12
ctcctttccc tcgacgcg 18
<210> 13
<211> 23
<212> DNA
<213> Artificial Sequence
<220>
<223> Description of Artificial Sequence: Synthetic DNA
<400> 13
ttgtgagttt taggtgaatg tgt 23
<210> 14
<211> 20
<212> DNA
<213> Artificial Sequence
<220>
<223> Description of Artificial Sequence: Synthetic DNA
<400> 14
ccctcctttc cctcaacaca 20

## Description

### Technical Field

The present invention relates to a method for detecting gastric cancer by detecting the inactivation of the human very low density lipoprotein receptor (human VLDLR) gene.

### Background Art

Every year, gastric cancer (GC) affects not less than 100,000 people in Japan. The male-to-female ratio of gastric cancer cases is approximately 2:1. The number of the male cases is larger than that of the female cases. Gastric cancer is the leading cancer for men. The number of annual deaths by gastric cancer is approximately 50,000 in Japan, accounting for 16% of the number of deaths from all cancers. Based on studies in the past, it is considered that genetic alterations sequentially occur in gastric tissue cells over the course of cell differentiation and cell growth, resulting in malignant transformation of the cells. However, the forms of such genetic alterations that induce gastric tissue cell malignant transformation have not been elucidated. Thus, there have been no methods for detecting gastric cancer or methods for examining the malignancy of gastric cancer.

Upon gastric cancer examination, abdominal x-ray examination, gastric endoscopy, a blood pepsinogen test, a tumor marker test, and/or the like are carried out. Gastric endoscopy (the only method by which definitive diagnosis can be made) has been widely performed. However, most of above examples can be regarded as tools for diagnostic imaging and early diagnosis. Thus, such examples are not intended to be applied to methods of definitive diagnosis of cancer, cancer typing, cancer staging, or determination of the presence or absence of cancer metastasis.

In addition, the method described in JP Patent Publication (Kohyo) No. 2005-514051 A or the like has been known as a method for detecting gastric cancer with a focus on genes. However, this method focuses on alterations in a gene expression profile. The measurement of a gene expression profile means the measurement of the amount of a plurality of mRNAs. mRNA is very unstable and likely to be degraded. In addition, the dynamic expression range of mRNA is wide, resulting in poor reproducibility of the measurement of mRNA with the use of a microarray system or the like. Therefore, highly stable and reproducible techniques for such measurement have been awaited.

Comparative genomic hybridization (CGH) is a method for detecting gene amplification on the genome with high sensitivity and gqod efficiency. With the use of such CGH array method, the present inventors identified a group of genes that are amplified in gastric cancer cells, including an SDC1 gene, a DNMT3A gene, an MLH1 gene, and a CTNNB1 gene, such genes being amplified at chromosomes 2p24.1, 2p23.3, 3p22.3, and 3p22.1, respectively (Takada H., Imoto I., Tsuda H., Sonoda I., Ichikura T., Mochizuki H., Okanoue T., and Inazawa J.: Screening of DNA copy-number aberrations in gastric cancer cell lines by array-based comparative genomic hybridization, Cancer Sci. 2005 Feb, 96(2), 100-10).

In general, in addition to the amplification of a plurality of genes as described above, deletion of cancer-suppressing gene has been reported to be involved in a mechanism of malignant transformation on a genetic level. However, identification of the deletion of a specific gene is very time- and labor-consuming with the use of general methods such as positional cloning. Thus, it is difficult to find a gene of interest. Further, it is also difficult to detect the deletion of a genomic DNA that is less than 5 to 10 Mb in size by the CGH method with the use of usual metaphase chromosomes ([Non-Patent Document 2] Bentz, M., Plesch, A., Stilgenbauer, S., Dohner, H., and Lichter, P.: Minimal sizes of deletions detected by comparative genomic hybridization, Genes Chromosomes Cancer, 172-175, 1998; and Kirchhoff, M., Gerdes, T., Maahr, J., Rose, H., Bentz, M., Dohner, H., and Lundsteen, C.: Deletions below 10 megabase pairs are detected in comparative genomic hybridization by standard reference intervals., Genes Chromosomes Cancer, 410-413, 1999). For instance, in the cases of cancer-suppressing genes such as DPC4/SMAD4, RB1, PTEN, p16-INK4A, and RASSF1, their homozygous deletion regions were narrowed down by a chromosome mapping method. Ultimately, cancer-suppressing genes of interest were identified.

### Disclosure of the Invention

It is an objective of the present invention to provide a highly stable and reproducible method for detecting gastric cancer, by finding a novel gastric cancer-related gene and detecting the inactivation of such cancer-related gene. It is another objective of the present invention to provide a method for early detection of gastric tissue cell malignant transformation or diagnosis of the malignancy of gastric cancer on a genetic level. Further, it is another objective of the present invention to provide a method for selecting an anticancer substance based on the mechanism of the above detection.

The present inventors increased the levels of sensitivity and accuracy of the CGH method so as to develop a high-throughput CGH method. They succeeded in identifying a cancer-suppressing gene which suppresses gastric tissue cell malignant transformation; namely, a human very low density lipoprotein receptor (VLDLR) gene (hereafter to be referred to as the "human VLDLR gene" in some cases), by selecting 800 types of BAC/PAC DNAs loaded on a CGH array. Then, they thoroughly elucidated the cause of the inactivation of the human VLDLR gene that induces gastric tissue cell malignant transformation. Accordingly, they have found that such gene inactivation is induced by (1) deletion of such gene and (2) methylation of CpG islands of such gene. These findings have led to the completion of the present invention.

That is, in accordance with the present invention, a method for detecting gastric cancer, comprising detecting malignant transformation of a gastric tissue cell by detecting the inactivation of the human VLDLR gene in the gastric tissue cell, is provided.

Preferably, genomic DNA, cDNA, or mRNA of the human VLDLR gene is used to detect the inactivation of the gene.

Preferably, the inactivation of the human VLDLR gene is the inactivation caused by deletion of the gene. Further preferably, the deletion of the human VLDLR gene is the homozygous deletion of the gene.

Preferably, the inactivation of the human VLDLR gene is the inactivation caused by methylation of CpG islands of the gene.

Preferably, the inactivation of the human VLDLR gene in a gastric tissue cell is detected using a DNA chip method, the Southern blot method, the Northern blot method, the real-time RT-PCR method, the FISH method, or the CGH method.

### Brief Description of the Drawings

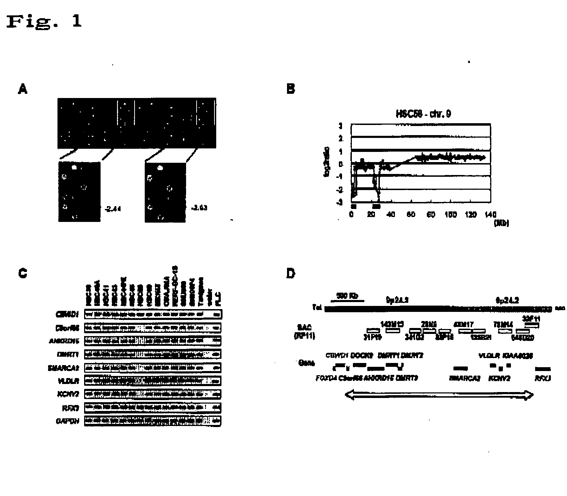

Fig. 1A shows an image of a typical CGH array (spotted in duplicate) of an HSC58 cell line. A remarkable decrease in the copy number ratio (log2 ratio) derived from RP11-48M17 at 9p24.2-24.3 was detected as a clear red signal. Fig. 1B shows a representative copy number profile for chromosome No. 9 in the HSC58 cell line. Red and blue bars represent candidate spots showing homozygous deletion patterns of 9p24.2-24.3 and 9p21.2-21.3, respectively. Fig. 1C shows an image obtained by genomic PCR experiments involving a GAPDH gene, control genes (based on genetic functions), and typical genes existing in the proximity of the site of homozygous deletion at 9p24.2-24.3 with the use of a plurality of gastric cancer cell lines such HSC58 (PLC: normal peripheral lymphocyte). Fig. 1D shows a map of 9p24.2-24.3, including a homozygous deletion region in an HSC58 cell line. Horizontal white bars represent BACs spotted on an array. A white horizontal arrow indicates the homozygous deletion region in an HSC58 cell, which was determined by genomic PCR. Among 13 genes found in the proximity of the region, those experiencing homozygous deletion in an HSC58 cell line and those remaining in the same are represented by black and gray bars, respectively. Sites and transcriptional directions of the genes are shown in fig. 1D.

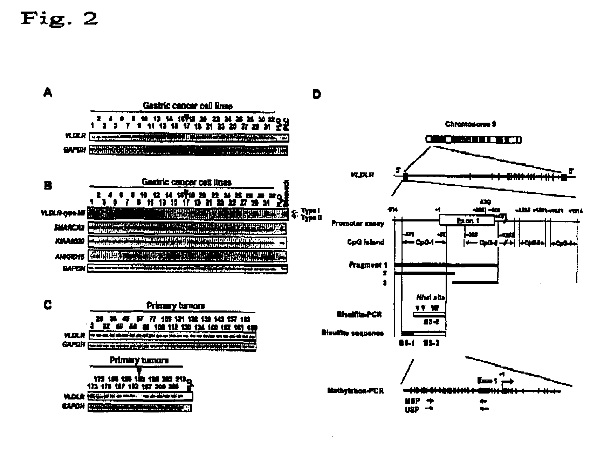

Fig. 2A shows results of genomic PCR analysis for detection of homozygous deletion (wedge sign) of VLDLR in gastric cancer cell lines (1: MKN2; 2: MKN7; 3: MKN28; 4: MKN45; 5: MKN74; 6: KATO-III; 7: OKAJIMA; 8: OCUM-1; 9: HSC39; 10: HSC40A; 11: HSC41; 12: HSC42; 13: HSC43; 14: HSC44PE; 15: HSC45; 16: HSC57; 17: HSC58; 18: HSC60; 19: HSC64; 20: NUGC-2; 21: NUGC-3; 22: NUGC-4; 23: RERF-GC-1B; 24: AZ-521; 25: SNU216; 26: SNU484; 27: SNU601; 28: SNU638; 29: SNU668; 30: SNU719; 31: SH101P4; and 32: Takigawa). Fig. 2B shows results of RT-PCR analysis for detection of the VLDLR expression in gastric cancer cell lines and normal gastric tissue. In fig. 2B, a wedge sign indicates a cell line shown in fig. 2A in which homozygous deletion was observed. It should be noted that almost complete inhibition of the VLDLR gene was observed in 9 (29.0%) out of 31 cell lines in which homozygous deletion of VLDLR was not observed, and an obvious decrease in the VLDLR expression was observed in 7 (22.6%) out of 31 cell lines compared with normal gastric tissue. Fig. 2C shows results of genomic PCR analysis for detection of 1 (indicated by a wedge sign) out of 39 LCM-treated primary gastric cancers, which were examined in terms of homozygous deletion of VLDLR. Fig. 2D shows a schematic diagram illustrating 4 CpG islands (in total) which were identified using the CPGPLOT (http://www.ebi.ac,uk/eMboss/cpgplot/). 4 vertical closed arrows represent 551-bp, 994-bp, 297-bp, and 274-bp CpG islands (CpG-1: -471 to +80; CpG-2: +269 to IVS+793; CpG-3: IVS+826 to IVS+1122; and CpG-4: IVS+1172 to IVS+1445), respectively. An outline box represents exon 1. The transcription initiation site is shown as "+1." Thick black lines represent fragments (fragments 1 to 3), which were examined by promoter assay. Horizontal gray bars represent regions (BS-1 and BS-2) that were examined by bisulfite-PCR analysis and bisulfite sequencing. Arrows shown in the bottom of fig. 2D indicate sites of MSP primers. CpG sites are represented by horizontal scales on the axis (enlarged view).

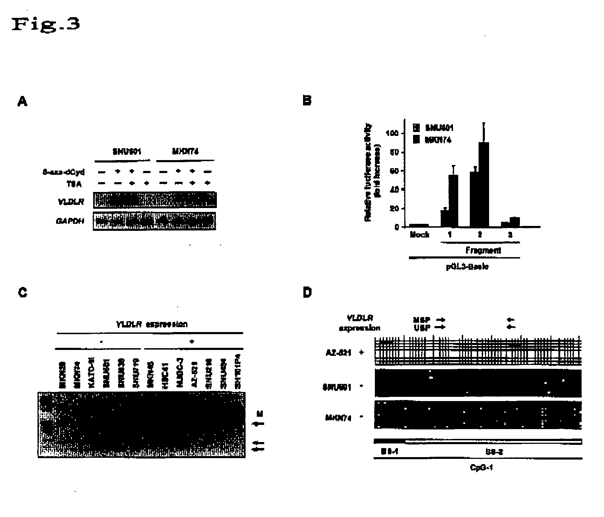

Fig. 3A shows results of typical RT-PCR analysis of the VLDLR expression in SNU601 and MKN74 cells, which were treated with 5-aza-dCyd (10 µM) for 5 days and/or TSA (10 ng/ml) for 24 hours, or left untreated. Fig. 3B shows promoter activities of regions with or without VLDLR CpG-1. An empty pGL3 basic vector (mock) and constructs each comprising a different one of 3 sequences (fragments 1 to 3) upstream or in the proximity of VLDLR exon 1 were separately introduced into SNU601 and MKN74 cells. Luciferase activity was normalized relative to that of an internal control. Data shown in fig. 3B contains mean values ± SEMs of triplicate experiments of 3 different constructs, Fig. 3C shows typical results of bisulfite-PCR analysis for VLDLR BS-2, which was digested with *Hha*I in gastric cancer cell lines. Arrows indicate fragments that were digested with restriction enzymes in a specific manner at sites recognized as methylated CpGs. Fig. 3D shows results of bisulfite sequencing of VLDLR CpG-1. Specifically, fig. 3D shows results of examination with the use of a VLDLR-expressing cell line (+) and a VLDLR-nonexpressing cell line (-). With the use of two fragments (BS-1 and BS-2), all of the 71 CpG sites were subjected to sequencing. Squares represent CpG sites (white square: unmethylated site; and black square: methylated site).

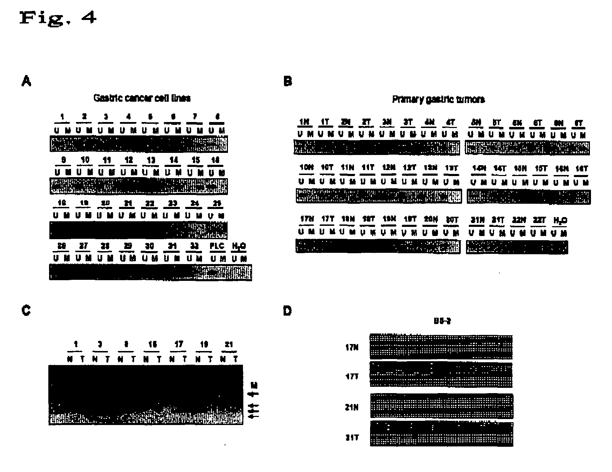

Fig. 4A shows typical results of MSP analysis of VLDLR CpG-1 in gastric cancer cell lines. As with the case of fig. 2A, the numbers in fig. 4A represent different GC cell lines. With the use of primers specific to unmethylated (U) and methylated (M) DNAs, amplification reactions were simultaneously performed. Fig. 4B shows typical results of MSP analysis of VLDLR CpG-1 with the use of primary gastric cancer tissues (T) and relevant non-gastric tissues (N). Fig. 4C shows typical results of bisulfite-PCR analysis of VLDLR BS-2 digested with *Hha*I with the use of samples of 7 primary gastric cancer patients. The results of MSP analysis indicate methylation in tumor tissues. In fig. 4C, arrows indicate fragments digested with restriction enzymes in a specific manner at sites recognized as methylated CpGs. Based on Fig. 4D, it is understood that the methylation condition of VLDLR BS-2 is determined via bisulfite sequencing of a pair of two typical gastric cancer samples (N: normal tissue; and T: tumor tissue).

### Best Mode for Carrying Out the Invention

Hereafter, embodiments of the present invention will be described in greater detail.

The method for detecting gastric cancer of the present invention comprises detecting the malignant transformation of a gastric tissue cell by detecting the inactivation of the human VLDLR. gene in the gastric tissue cell.

A preferred example of a gastric tissue cell subjected to detection of the inactivation of the human VLDLR gene is a biopsy tissue cell specimen obtained from a test subject. Such tissue cell specimen may be a gastric tissue cell of a healthy volunteer or a cancer tissue cell of a gastric cancer patient. In practice, examples of a target tissue specimen that can be used include mainly (1) a tissue obtained from a lesion in which suspected malignant transformation of gastric tissue is observed by endoscopy or the like and (2) a cancer tissue that has been confirmed to be derived from gastric cancer and thus must be subjected to determination of malignancy or the stage of gastric cancer.

In a case in which the inactivation of the human VLDLR gene is observed in "a tissue obtained from a lesion in which suspected malignant transformation of gastric tissue is observed by endoscopy or the like" by the detection method of the present invention, it is understood that such lesion tissue will reach (or has reached) the state of malignant transformation so that the level of malignancy of the disease will increase. Thus, there is a demonstrated urgent need for implementation of a full-scale therapy (e.g., elimination of a lesion via a surgery or the like and full-scale chemotherapy). In addition, in a case in which the inactivation of the human VLDLR gene is observed in "a cancer tissue that has been confirmed to be derived from gastric cancer and thus must be subjected to determination of malignancy or the stage of gastric cancer," it is also understood that the level of malignancy of the disease will increase. Thus, there is a demonstrated urgent need for implementation of full-scale therapy (e.g., elimination of a lesion via a surgery or the like and full-scale chemotherapy). A gastric tissue cell collected as a specimen may be subjected to necessary treatment such as preparation of DNA or RNA from the collected tissue followed by the detection method of the present invention.

As described above, in accordance with the detection method of the present invention, it is possible to identify malignant transformation (and particularly, the initiation of malignant transformation) of a gastric tissue cell by detecting the inactivation of the human VLDLR gene in the cell. Examples of forms of such inactivation of the human VLDLR gene include (1) human VLDLR gene deletion and (2) a decrease in the expression level of the human VLDLR gene in a case in which deletion of the gene is not observed.

### (1) Human VLDLR gene deletion

Representative examples of a method for directly detecting the aforementioned human VLDLR gene deletion include the CGH (comparative genomic hybridization) method and the FISH (fluorescence *in situ* hybridization) method, In this embodiment, according to the detection method of the present invention, it is possible to detect a region lacking the human VLDLR gene by labeling BAC (bacterial artificial chromosome) DNA, YAC (yeast artificial chromosome) DNA, or PAC (phage artificial chromosome) DNA (hereafter to be collectively referred to as BAC DNA or the like in some cases), each of which has the human VLDLR gene, followed by FISH. Specific examples of BAC DNA having the human VLDLR gene include RP-11-671F16, RP11-320E16, RP11-578M2, RP11-719M6, and RP11-941D23.

It is preferable and practical to carry out the method in the above embodiment with the use of a genomic DNA-immobilized matrix.

The amount of BAC DNA or the like obtained in a conventional manner is so small that a large number of genomic DNA-immobilized matrices cannot be produced for practical application. Thus, it is necessary to obtain gene amplification products of such DNA. (A gene amplification process for this purpose is referred to as "infinite amplification" in some cases.) Upon infinite amplification, BAC DNA or the like is first digested with a four-nucleotide recognition enzyme such as *Rsa*I, *Dpn*I, *Hae*III, or the like, followed by ligation with the addition of an adapter.

An adapter comprises oligonucleotides having 10 to 30 nucleotides and preferably 15 to 25 nucleotides. Double strands of such adapter have sequences complementary to each other. After annealing, the 3' end of one of the oligonucleotides, at which a blunt end is formed, must be phosphorylated. Next, a primer having a sequence identical to the other oligonucleotide of the adapter is used for amplification via PCR (polymerase chain reaction). Thus, infinite amplification can be carried out. Meanwhile, it is also possible to use, as a detection probe, an aminated oligonucleotide comprising 50 to 70 nucleotides, which is inherent to BAC DNA or the like.

BAC DNAs or the like subjected to infinite amplification are immobilized on a matrix and preferably on a solid matrix. Accordingly, a desired DNA-immobilized matrix can be produced. Such solid matrix may be made of glass, plastic, or the like and may be in the form of a membrane, a 3-D array, or the like. A glass matrix such as an object glass is preferable. Further preferably, a solid matrix made of glass or the like is coated via adhesion with poly-L-lysine, aminosilane, gold, aluminium, or the like.

The concentration of DNA subjected to infinite amplification to be spotted on a matrix is preferably 10 pg/µl to 5 µg/µl and more preferably 1 ng/µl to 200 ng/µl. The amount of the same to be spotted on the matrix is preferably 1 nl to 1 µl and more preferably 10 nl to 100 nl. In addition, the size and the shape of each spot that is immobilized on the matrix are not particularly limited. In terms of size, such spot may have a diameter of 0.01 to 1 mm, for example. In addition, the shape of such spot may be a circle or elipse from an overhead view. The thickness of a dry spot is not particularly limited; however, it may be 1 to 100 µm. Further, the number of spots is not particularly limited; however, it may be 10 to 50,000 spots and more preferably 100 to 5,000 spots on the matrix used. DNAs are spotted singly to quadruplicate. However, preferably, DNAs are spotted in duplicate or triplicate.

Regarding preparation of dry spots, it is possible to produce dry spots by, for example, spotting BAC DNAs or the like subjected to infinite amplification on a matrix with the use of a spotter, forming a plurality of spots thereon, and drying the spots. Examples of a spotter that can be used include an inkjet printer, a pin-array printer, and a bubble jet (trademark) printer. Among them, an inkjet printer is preferably used. For instance, GENESHOT (NGK INSULATORS; Nagoya, Japan) or the like can be used.

As described above, it is possible to produce a desired DNA-immobilized matrix by immobilizing BAC DNAs or the like subjected to infinite amplification onto a matrix, and preferably, onto a solid matrix.

In addition, an example of a means of directly detecting the aforementioned human VLDLR gene deletion is the Southern blot method. The Southern blot method is a method for detecting the existence of the human VLDLR gene in a specimen by separating and immobilizing genomic DNA obtained from the specimen and detecting hybridization of such genomic DNA with the human VLDLR gene. The nucleotide sequence and the amino acid sequence of the human VLDLR gene, which have been known to the public, are set forth in SEQ ID NOS: 1 and 2 in the Sequence Listing, respectively.

### (2) Decrease in the expression level of the human VLDLR gene in a case in which deletion of the gene is not observed

With the use of the method described in this embodiment, it is possible to detect a decrease in the expression level of the human VLDLR gene by quantifying the amount of polynucleotide or human VLDLR protein derived from the human VLDLR gene (genomic DNA) and using the quantified value as an index. Examples of such polynucleotide include mRNA transcribed from the human VLDLR gene and cDNA obtained with the use of such mRNA as a template.

In addition, the above quantified value can be obtained by, for example, preparing an antibody against a human VLDLR protein according to a conventional method and quantifying the amount of human VLDLR protein in a specimen with the use of such antibody. However, since a "decrease" in the gene expression level is detected by the detection method of the present invention, it is necessary to solve problems such as the presence of backgrounds in order to designate the amount of protein as an index for such negative detection. Thus, such detection cannot be efficient. Therefore, as a means for detection in this embodiment, it is preferable to use a method for detecting the amount of polynucleotide derived from the human VLDLR gene (genomic DNA). Examples of such method for detecting the amount of polynucleotide will be described below.

According to the detection method of the present invention in the most typical embodiment, cDNA is detected using, as a template, mRNA of a human VLDLR protein in a specimen, mRNA that exists in the specimen is indirectly detected, and the expression level of human VLDLR gene is obtained. (Hereafter, the means for detection in this embodiment is referred to as a method for detecting VLDLR mRNA in some cases. Examples of such detection method in this embodiment include the real-time RT-PCR method described below).

Regarding such method for detecting VLDLR mRNA, mRNA is typically selected from among total RNAs of a specimen according to a conventional method (e.g., a method using oligodT). Then, cDNA encoding a human VLDLR protein is amplified by a gene amplification method (e.g., the RT-PCR method) with the use of the obtained mRNA. Such method enables gene amplification with the use of mRNA as a template, a nucleotide chain corresponding to the known nucleotide sequence of the human VLDLR gene as a primer, and thermostable DNA polymerase. In addition, the presence or absence of the resulting gene amplification product or the amount of the same obtained by such gene amplification operation is detected. Thus, the existence of mRNA of human VLDLR protein in a specimen can be detected, preferably in a real-time manner.

The real-time RT-PCR method is a method for measuring amplification of a gene of interest in a time-dependent (real-time) manner via a reverse transcription reaction and a polymerase chain reaction. In accordance with such method, mRNA that serves as a template can be quantified based on amplification efficiency. Such quantification is carried out using a fluorescent dye. There are two ways of quantification. Specifically, the following methods have been known to the public: a method using a dye (e.g., SYBR green) that causes the expression of fluorescence when such dye is intercalated into double-strand DNA in a specific manner and a method using a probe in which a fluorescent dye is allowed to bind to an oligonucleotide that is specific to a DNA sequence to be amplified.

In addition, an additional gene amplification operation (PCR or the like) with the use of a thermostable DNA polymerase is carried out using, as a template, cDNA encoding a human VLDLR protein, which has been amplified as described above. The presence or absence of the resulting gene amplification product or the amount of the same is detected. Thus, the existence of mRNA of the human VLDLR protein in a specimen can be further accurately detected. (A primer used for gene amplification for the additional (2^{nd}) gene amplification operation must be a nucleotide chain that corresponds well to the inside of the human VLDLR gene compared with a primer used for the 1^{st} gene amplification operation.)

A DNA chip method is an example of a method for quantifying mRNA expressed in a cell specimen. For instance, a synthetic oligonucleotide having a sequence that is inherent to the human VLDLR gene is immobilized on a matrix (which is substantially identical to a matrix used in the aforementioned CGH method) (or cDNA can be immobilized thereon). Then, labeling is carried out when cDNA is synthesized with the use of a reverse transcriptase and RNA prepared from the specimen. Thereafter, the thus labeled cDNA is hybridized with the synthetic oligonucleotide on the matrix such that the expression level of mRNA can be measured by scanning the amount of the label of cDNA binding to the oligonucleotide.

The Northern blot method is a method for detecting the expression of the human VLDLR gene in a specimen, wherein mRNA obtained from the specimen is separated and immobilized and hybridization of the mRNA with a synthetic oligonucleotide that has a sequence specific to the human VLDLR gene is detected. (It is also possible to use cDNA instead of such synthetic oligonucleotide.)

In addition to human VLDLR gene deletion, the inactivation of the human VLDLR gene due to CpG island methylation is also a main reason for decreases in the expression level of the gene. Such reason is simply described below.

It has been reported that transcriptional inactivation occurs when a CpG-rich promoter and an exon region are densely methylated (Bird, A. P. & Wolffe, A. P.: Methylation-induced repression-belts, braces, and chromatin, Cell, 451-454, 1999). In the cases of cancer cells, CpG islands are frequently and densely methylated compared with other regions, and thus hypermethylation of a promoter region is deeply involved in the inactivation of a cancer-suppressing gene of a human cancer (Ehrlich, M., Jiang, G., Fiala, E., Dome, J. S., Yu, M. C., Long, T. I., Youn, B., Sohn, O. S., Widschwendter, M., Tomlinson, G. E., Chintagumpala, M., Champagne, M., Parham, D., Liang, G., Malik, K., and Laird, P. W.: Hypomethylation and hypermethylation of DNA in Wilms tumors, Oncogene, 6694-6702, 2002).

As described below, a portion of human VLDLR gene exon 1 and CpG islands located upstream of the portion were found to have promoter activities. In addition, the extent of methylation of the CpG islands strongly correlated with inhibition of the expression of the human VLDLR gene in some gastric cancer cells. In addition, it was possible to demetylate such CpG islands by culturing such gastric cancer cells in the presence of 5-azadeoxycytidine (5-aza-dC) serving as a demethylating reagent. As a result, it was possible to recover the expression level of the human VLDLR gene. Based on the above results, it has been revealed that highly dense methylation (hypermethylation) of CpG islands is a cause of frequently occurring inhibition of the expression of a cancer-suppressing gene in gastric cancer cells.

The present invention is hereafter described in greater detail with reference to the following examples, although the technical scope of the present invention is not limited thereto.

### Examples

### [Example 1]

With the use of genome database websites of the National Cancer for Biotechnology Information and the University of California Santa Cruz Biotechnology and results of BLAST searches concerning selected DNAs, 800 different BAC/PAC clones each having a gene playing a important role in malignant transformation and cancer cell growth or a sequence tagged site marker were selected.

BAC and PAC DNAs were digested with *Dpn*I, *Rsa*I, and *Hae*III, followed by ligation with adapter DNAs. Next, PCR was performed twice with the use of primers having the sequences of the adapters. One end of the pairs of primers had an aminated 5' end. This process is referred to as "infinite amplification." The thus obtained DNAs are defined as "DNAs obtained by infinite amplification." DNAs obtained by infinite amplification were printed in duplicate on an Oligo-DNA Microarray (Matsunami Glass; Osaka, Japan) with the use of an inkjet spotter (GENESHOT, NGK INSULATORS; Nagoya, Japan) such that they were covalently bound to the microarray.

### [Example 2]

In order to detect a novel homozygous deletion in the case of gastric cancer, CGH array analysis was carried out with the use of genomic DNAs prepared from 32 different gastric cancer cells and the CGH array of Example 1.

In addition, genomic DNAs of healthy male volunteers were labeled with Cy5 so as to be used as controls. Genomic DNAs prepared from the aforementioned gastric cancer cells were labeled with Cy3 so as to be used as test sample DNAs. Specifically, genomic DNAs (0.5 µg each) digested with *Dpn*I were labeled by nick translation in the presence of 0.2 mM dATP, 0.2 mM dTTP, 0.2 mM dGTP, 0.1 mM dCTP, and 0.4 mM Cy3-dCTP (used for gastric cancer cells) or 0.4 mM Cy5-dCTP (used for normal cells). Cy3- and Cy5-dCTPs were obtained from Amersharn Biosciences (Tokyo, Japan). Both labeled genomic DNAs were precipitated in the presence of Cot-1DNA (Invitrogen) with the addition of ethanol. The resultant was dissolved into 120 µl of a hybridization mixture (50% formamide, 10% dextransulfate, 2 x SSC (1 x SSC: 150 mM NaCl/15 mM Sodium Citrate), and 4% sodiumdodecylsulfate; pH 7). The resulting solution was incubated at 37°C for 30 minutes and added to the CGH array that was placed in a hybridization chamber. The array was simultaneously subjected to incubation at 37°C and agitation at a rate of 3 rpm (rounds per minute) for 48-72 hours. Subsequently, the CGH array was washed in a 50% formamide/2 x SSC (pH 7.0) solution at 50°C for 15 minutes. Then, the array was washed in 2 x SSC/0.1% SDS at 50°C for 15 minutes. Further, the array was washed with the use of a 0.1 M phosphate buffer (pH 8) containing 0.1% Nonidet P-40 at room temperature for 15 minutes, followed by air drying. Then, the CGH array was subjected to monitoring of Cy3-derived fluorescence and Cy5-derived fluorescence with the use of a GenePix 4000B scanner (Axon Instruments, CA, U.S.A.). The obtained results were analyzed using the GenePixPro 4.1 imaging software (Axon Instruments). The mean value of Cy3-derived fluorescence intensities and that of Cy5-derived fluorescence intensities were adjusted to the same value. Then, the Cy3/Cy5 ratio was obtained. Note that, if a genome is normal, such ratio value is 1. When the ratio value was not less than 1.32, it was determined that genome amplification was observed. When the ratio value was not less than 4, it was determined that significant amplification was observed. When the ratio value was not more than 0.75, it was determined that the possibility of heterozygous deletion in the genome was significantly high. Also, when the ratio value was not more than 0.25, the possibility of homozygous deletion was significantly high.

Accordingly, the human VLDLR gene at chromosome 9p24.2 was found to be deleted in a gastric cancer cell (HSC58). In such case, the Log2 ratio was -2.63. [Fig. 1A shows a representative array image obtained by carrying out CGH analysis on the CGH array described in Example 1 with the use of the genomic DNA of HSC58 (a sample cell). A decrease in the copy number ratio of the human VLDLR gene (chromosome 9p24.2-24.3 region) was represented by a clear signal (Log2 ratio = -2.63) (indicated by an arrow in the right-side enlarged view).]

Also, other cell lines were examined. Accordingly, human VLDLR gene homozygous deletion was not observed in 31 different gastric cancer cells, [Fig. 2A shows results of detection of human VLDLR gene homozygous deletion in gastric cancer cells. The results of PLC were derived from DNA of a healthy volunteer. PCR results for the GAPDH (Glyceral dehyde-3-phosphate dehydrogenase) gene were used as control results.] Further, human VLDLR gene homozygous deletion was observed in 1 (indicated by a wedge sign in fig. 2C) out of 39 primary gastric cancer samples subjected to laser-captured microdissection (LCM). [Fig. 2C shows results indicating human VLDLR gene homozygous deletion in primary gastric cancers treated by laser-captured microdissection (LCM). A wedge sign (fig. 2C) indicates a cell line in which homozygous deletion was detected, PCR results for the GAPDH gene were used as control results.]

### [Example 3]

Two types of primers were designed based on the sequence of the human VLDLR gene in the gastric cancer cells. mRNAs derived from both primer regions were detected by RT-PCR.

As a result, it was impossible to detect mRNAs derived from cell lines in which homozygous deletion was observed. Further, RT-PCR products were not detected from 9 cell lines in which homozygous deletion was not observed (among 32 cell lines). In the cases of 7 cell lines, the expression level obviously decreased compared with normal gastric tissue. [Fig. 2B shows results of RT-PCR analysis for detection of the human VLDLR expression in gastric cancer cells. In fig. 2A, cell lines in which homozygous deletion was observed are indicated by arrows in a similar manner. In 9 cell lines in which homozygous deletion was not observed (among 31 cell lines), the human VLDLR mRNA expression disappeared at a frequency of 29.0% and the expression level decreased at a frequency of 22.6%.] Thus, in addition to gene deletion on the genome, other mechanisms were thought to make it impossible to detect human VLDLR mRNA. Therefore, such mechanisms were further examined.

### [Example 4]

CpG island methylation is a mechanism suppressing gene expression. CpG islands of the human VLDLR gene were analyzed by the CpGPLOT. As a result, 4 regions were identified as being located around the human VLDLR gene exon 1. CpG sites in such regions and CpG islands in which CpG sites densely exist are shown in fig. 2D. [Fig. 2D is an illustrative map of CpG islands located around the human VLDLR gene exon 1. An outline box represents exon 1. The transcription initiation site is indicated as "+1." CpG islands which were identified by the CpGPLOT (http://www.ebi.ac.uk/emboss/cpgplot/) are indicated by horizontal arrows. CpG islands range from positions -514 to +1914 of the human VLDLR gene. Regions analyzed by promoter assay (fragments 1 to 3), a region subjected to bisulfite-PCR analysis, and a region subjected to bisulfite sequencing are represented by horizontal bars.]

In order to examine promoter activity derived from the identified CpG islands of the human VLDLR gene, 3 different fragments were prepared from the CpG islands and the proximity thereof (fig. 2D: fragments 1-3). Then, the fragments were separately inserted into a luciferase reporter plasmid (pGL3-Basic vector: Promega, WI, U.S.A.). Each plasmid was transiently cotransfected together with an internal control vector (pRL-hTK, Promega) into an SNU601 cell and an MK704 cell. The reporter assay system used was based on a method for measuring a light emitting substance in which messenger RNA of firefly luciferase is expressed, the mRNA is converted into a protein, and the thus obtained protein acts as an enzyme having activity. With the use of a pOL3-Basic vector (Promega), a luciferase gene was placed downstream of the VLDLR gene. Then, constructs containing VLDLR gene fragments 1 to 3 (promoter analysis regions) were introduced into SNU601 and MK704 cells, in which VLDLR gene expression was not observed. Subsequently, the enzyme activity of each construct was measured (fig. 3B). The fragment 2 showed a high level of luciferase activity in both cells. Accordingly, it has been revealed that a basic unit of a transcriptional activation domain of the VLDLR gene is contained in the fragment 2.

According to a method for detecting gene mutation, unmethylated cytosine (C) in genomic DNA is converted into uracil (U) with sodium bisulfite treatment. However, methylated cytosine is structurally stable, so it is not influenced by a reaction caused by such treatment and is not converted into uracil. The presence of methylated DNA can be identified in a manner such that: PCR detection is performed with the use of such methylated cytosine as a template; and differences between the sequence of altered cytosine and the sequence of an amplified DNA product derived from unmethylated cytosine are analyzed based on the occurrence or nonoccurrence of restriction enzyme cleavage with the use of a restriction enzyme susceptible to methylation (COBRA method: an experimental method combining the bisulfite-PCR method and the restriction enzyme cleavage method). Thus, methylation that occurred in the proximity of VLDLR gene exon 1 was detected by the COBRA method (fig. 3C). Each of MKN28, MKN74, KATO-III, SNU601, SNU638, and SNU719 cell lines with low expression levels of the VLDLR gene had a restriction enzyme (*Hha*I) cleavage site. As a result, 3 bands (indicated by arrows) derived from cleaved DNAs were observed by electrophoresis. Meanwhile, each of MKN45, HSC41, NUGC-3, AZ-521, SNU216, SNU484, and SH101P4 cell lines, in which VLDLR gene expression was observed, had no methylation-dependent restriction enzyme cleavage sites. As a result, a single band (M) was observed by electrophoresis.

The level of methylation of each CpG dinucleotide in CpG islands of the human VLDLR gene was analyzed by bisulfite sequencing. Accordingly, in the cases of cells (SNU601 and MKN74) in which homozygous deletion was not observed, the levels of methylation of CpG islands of the human VLDLR gene were particularly high. Meanwhile, in the cases of a cell (AZ-521) in which the human VLDLR gene was expressed, methylation did not occur. [Fig. 3D shows results of bisulfite genomic sequencing of CpG islands of the human VLDLR gene. Given that the transcription initiation site is represented by "+1," CpG islands range from positions +471 to +1914. In this region, there are 71 CpG sites, which are represented by grids. Outline grids represent unmethylated CpG sites and black grids represent methylated CpG sites. Cells used were AZ-521, which is a human VLDLR gene-expressing cell (indicated by expression (+)), and SNU601 and MKN74, each of which is a human VLDLR gene-nonexpressing cell (indicated by expression (-)).] Thus, it has been revealed that the human VLDLR gene expression is not detected or is detected at a very low level in the cases in which human VLDLR gene homozygous deletion occurs in a gastric cancer cell and in which the CpG islands of the human VLDLR gene are highly methylated. These two cases strongly suggest that there is a relationship between the human VLDLR gene expression and gastric tissue cell malignant transformation.

### [Example 5]

Methylation of CpG islands of the human VLDLR gene in gastric cancer cells was analyzed in the above Examples. In addition, it was examined whether or not a similar phenomenon would actually occur in gastric cancer tissue. As a result, methylation of CpG islands of the human VLDLR gene was found to occur in 7 primary gastric cancer cases. [Fig. 4C shows results of COBRA analysis of CpG islands of the human VLDLR gene in primary gastric cancers. "N" denotes non-cancer gastric tissue and "T" denotes primary gastric cancer tissue]. Based on the above results, it is predicted that the inactivation of the human VLDLR gene due to methylation of CpG islands of the human VLDLR gene plays an important role in malignant transformation of gastric tissue cell.

### [Example 6]

The human VLDLR gene expression was examined in the cases in which CpG islands of the human VLDLR gene in gastric cancer cells were demethylated. Specifically, gastric cancer cells were treated in the presence of 5-aza-dC (serving as a transmethylase inhibitor) at different concentrations for 5 days, followed by determination of the capacity of inducing human VLDLR mRNA. A cell in which human VLDLR mRNA was not detected even though human VLDLR gene deletion did not occur therein was subjected to a 5-aza-dC treatment. Accordingly, such treated cell was found to have the capacity of inducing human VLDLR mRNA [Fig. 3A shows results of analysis of human VLDLR gene expression in gastric cancer cells in the presence or absence of 5-aza-dC. RT-PCR was carried out with the use of two types of specific primers used for amplification of human VLDLR gene, followed by electrophoresis. The thus obtained bands are shown in fig. 3A. GAPDH was used as an internal control. The genomes used as templates were prepared after 5-day culture of cell species (SNU601 and MKN74) in the presence or absence of 5-aza-dC, such cell species not expressing the human VLDLR gene without homozygous deletion.]

### [Example 7]

It has been elucidated that gene expression inhibition due to CpG island methylation results from alternations in the chromosomal structure of the genome. Also, alterations in the chromosomal structure of the genome are caused by partial deacetylation of a histone protein that interacts with the genome so that such alterations influence gene expression. In this example, the relationship between CpG methylation and histone acetylation was examined using trichostatin A (TSA) serving as a histone deacetylase inhibitor and 5-aza-dC serving as a transmethylase inhibitor.

SNU601 and MKN74 cells in which CpG islands of the human VLDLR gene were densely methylated were incubated in the presence of TSA. Accordingly, the obtained human VLDLR gene expression level was equivalent to that obtained in the absence of TSA. [Fig. 3A shows the expression of VLDLR mRNA in gastric cancer cells (SNU601 and MKN74) in which the human VLDLR gene was not expressed and which were cultured in the presence of 5-aza-dC and TSA. Both cells were cultured in the presence of 5-aza-dC for 5 days, followed by culture in the presence of TSA or the presence of 5-aza-dC and TSA for 12 hours. Genomic DNAs prepared from both cells were used as templates. Then, RT-PCR was performed with the use of two types of specific primers used for amplification of human VLDLR gene, followed by electrophoresis. The thus obtained bands are shown in fig. 3A. GAPDH was used as an internal control.] When both cells were incubated in the presence of TSA and 5-aza-dC, there were no particular alterations in human VLDLR gene expression. The result suggests that CpG island methylation exclusively influences human VLDLR gene expression but does not relate to alterations in higher-order chromosomal structure.

### [Example 8]

When a method for detecting methylation of a promoter region is carried out, it is possible to convert unmethylated cytosine (C) in genomic DNA into uracil (U) via sodium bisulfite treatment. Also, it is possible to detect methylation of a specific site by PCR (methylation specific PCR (MSP) method) with the use of a pair of primers having uracil sites that correspond to the uracil sites of DNA to be examined, which have been altered via sodium bisulfite treatment. With the use of such method, samples of gastric cancer cell lines, clinical samples of cancer tissues, and clinical samples of normal neighboring tissues of the cancer tissues were analyzed using the MSP primer shown in fig. 2D (figs. 4A and 4B; U: unmethylated; M: methylated; N: normal; and T: tumor). The results by the MSP method for gastric cancer cell lines (MKN74: No. 5 and SNU601: No. 27) in which the VLDLR gene expression was not observed indicated methylation of promoter regions of both cell lines. The results for an AZ-521 (No. 24) cell in which the VLDLR gene expression was observed indicated no methylation. The obtained results were as expected. Upon methylation analysis of the clinical cancer tissue samples based on the MSP method, methylation was detected in 7 out of 20 samples, In addition, it was possible to analyze cancer tissue sites or normal neighboring tissue sites in 5 out of 7 samples. The results obtained from the 5 samples indicated that cancer-specific methylation was detected in cancer tissue sites and methylation was not detected in normal neighboring tissue sites. With the use of samples (17 and 21) in which such difference in terms of methylation was detected, methylation of VLDLR gene BS-2 region (see fig. 2D) was further analyzed by the bisulfite-sequence method (fig. 4D). In the cases of cancer samples 17 and 21 in which the difference in terms of methylation was detected, methylation was observed as expected (methylated sites are shown in black).

Primer sequences and PCR conditions used in the above Examples are listed in table 1.

**Table 1. Primer sequences and POR conditions for the VLDLR gene**

| **Method** | | **Forward primer** | **Reverse primer** | **Annealing (°C)** |
|---|---|---|---|---|
| Genomic PCR | | 5'-CCAGAGATATCAGTTGTAAGCA | 5'-AAAAGAGACTTGAGCTGCTGG | 60 |
| Reverse transcription -PCR | | 5'-CTAGTOAAOAACCTGAATQATQ | 6'-AAGAATOOCCATCCOOCACAA | 62 |
| Biaulfite-PCR | BS-1 | 5'-GAAATTGTAAGTATTTTTTAQQTQT | 5'-TACCTATCCTAATTTCCTAATCC | 58 |
| | BS-2 | 5'-GGATTAGGAAATTAOOATAGOTA | 5'-CCCACCCAACCACCTCCC | 60 |
| Methylation-specifle PCR | M | 5'-GAQTTTTAGGCGAAOGOGOGO | 5'-OTOOTTTOOCTCOACOCO | 60 |
| | U | 5'-TTGTGAGTTTTAGGTGAATQTQT | 5'-CCCTCOTTTCCCTCAACACA | 60 |

### Effect of the Invention

According to the present invention, it becomes possible to precisely understand signs of malignant transformation detected from a gastric tissue cell specimen and the stage or the malignancy of a cancer found in such specimen.

## Claims

1. A method for detecting gastric cancer, comprising detecting malignant transformation of a gastric tissue cell by detecting the inactivation of the human VLDLR gene in the gastric tissue cell.

2. The method for detecting gastric cancer according to claim 1, wherein genomic DNA, cDNA, or mRNA of the human VLDLR gene is used to detect the inactivation of the gene.

3. The method for detecting gastric cancer according to claim 1, wherein the inactivation of the human VLDLR gene is the inactivation caused by deletion of the gene.

4. The method for detecting gastric cancer according to claim 3, wherein the deletion of the human VLDLR gene is the homozygous deletion of the gene.

5. The method for detecting gastric cancer according to claim 1, wherein the inactivation of the human VLDLR gene is the inactivation caused by methylation of CpG islands of the gene.

6. The method for detecting gastric cancer according to claim 1, wherein the inactivation of the human VLDLR gene in a gastric tissue cell is detected using a DNA chip method, the Southern blot method, the Northern blot method, the real-time RT-PCR method, the FISH method, or the CGH method.
